# EUROPEAN PATENT APPLICATION

(11) **EP 4 180 514 A1**
(43) Date of publication of application: **17.05.2023**
(21) Application number: 21845347.0
(22) Date of filing: 09.07.2021
(51) Int. Cl.: C12N 5/02, C12N 5/0735, C12N 5/10, C12N 15/00

(54) **COMPOSITION FOR UNDIFFERENTIATED MAINTENANCE CULTURE OF PLURIPOTENT CELLS, MEDIUM FOR UNDIFFERENTIATED MAINTENANCE CULTURE OF PLURIPOTENT CELLS, MAINTENANCE CULTURE METHOD IN UNDIFFERENTIATED STATE OF PLURIPOTENT CELLS, AND METHOD FOR PRODUCING PLURIPOTENT CELLS**

(30) Priority: 20.07.2020 JP 2020123653
(71) Applicant: Aichi Medical University, Aichi 480-1195 (JP); Sumitomo Seika Chemicals Co., Ltd., Kako-gun, Hyogo 675-0145 (JP)
(72) Inventor: HATAYAMA Naoyuki, Nagakute-shi, Aichi 480-1195 (JP); NAITO Munekazu, Nagakute-shi, Aichi 480-1195 (JP); HIRAI Shuichi, Nagakute-shi, Aichi 480-1195 (JP); FUKUSHIGE Kaori, Nagakute-shi, Aichi 480-1195 (JP); OYAMA Shunji, Osaka-shi, Osaka 541-0041 (JP)
(74) Representative: Hoffmann Eitle
(86) International application number: PCT/JP2021/025973
(87) International publication number: WO 2022/019152

(57) **Abstract**

The present invention provides a composition for pluripotent cell undifferentiated state maintenance culture and a culture method using the same, which are capable of improving the growth rate of pluripotent cells or reducing the frequency of cellular death of pluripotent cell in the pluripotent cell maintenance culture in an undifferentiated state. A composition for pluripotent cell undifferentiated state maintenance culture includes microbubbles.

## Description

### TECHNICAL FIELD

The present invention relates to a composition for pluripotent cell undifferentiated state maintenance culture, a medium for pluripotent cell undifferentiated state maintenance culture, a method for pluripotent cell undifferentiated state maintenance culture, and a method for producing pluripotent cells.

### BACKGROUND ART

In regenerative medicine, an attempt has been made to induce a differentiation of cells having pluripotency (pluripotent cells) into target cells (Non-Patent Literature 1) and treat them using the obtained cells or cell populations (therapeutic cells).

### Citation List

### Patent Literature

### Non-Patent Literature

Non-Patent Literature 1: Hyunjin Choi et al., "Present status of large-scale culture method of pluripotent stem cell: Differentiation induction of human iPS cells with dialysis suspension culture," 2019, Organ Biology, VOL.26, NO.2, pp. 85-94

### SUMMARY OF INVENTION

### Technical Problem

When the therapeutic cells are applied to human therapy, a large number of pluripotent cells are required. Since pluripotent cells are capable of self-renewal, it is possible to increase the number of pluripotent cells by performing maintenance culture in an undifferentiated state. Hence, the method for pluripotent cell undifferentiated state maintenance culture by suppressing the differentiation ability of pluripotent cells has been studied.

Therefore, there is a demand for a method capable of growing pluripotent cells more efficiently while maintaining the undifferentiated state of pluripotent cells.

With the foregoing in mind, it is the object of the present invention to provide a composition for pluripotent cell undifferentiated state maintenance culture and a culture method using the same that are capable of improving the growth rate of pluripotent cells or reducing the frequency of cellular death of pluripotent cells in the pluripotent cell maintenance culture in an undifferentiated state.

### A Solution to the Problem

In order to achieve the above object, the present invention provides a composition for pluripotent cell undifferentiated state maintenance culture (hereinafter, also referred to as a "maintenance composition"), including microbubbles.

The present invention also provides a medium for pluripotent cell undifferentiated state maintenance culture (hereinafter, also referred to as a "maintenance medium"), including the composition for pluripotent cell undifferentiated state maintenance culture according to the present invention.

The present invention also provides a method for pluripotent cell undifferentiated state maintenance culture (hereinafter, also referred to as a "maintenance culture method"), including pluripotent cell maintenance culturing in an undifferentiated state in the presence of microbubbles.

The present invention also provides a method for producing pluripotent cells (hereinafter, also referred to as a "production method"), including producing pluripotent cells by performing pluripotent cell maintenance culture in an undifferentiated state, wherein the production is conducted by the method for pluripotent cell undifferentiated state maintenance culture according to the present invention.

### Advantageous Effects of the Invention

According to the present invention, the growth rate of pluripotent cells can be improved, or the frequency of cellular death of pluripotent cells can be reduced, as compared to a composition for pluripotent cell undifferentiated state maintenance culture including substantially no microbubbles.

### BRIEF DESCRIPTION OF DRAWINGS

[FIG. 1] FIG. 1 is a schematic diagram showing an apparatus for producing a maintenance composition in Example 1.
[FIG. 2] FIG. 2 is a diagram showing a protocol of the maintenance culture in Example 1.
[FIG. 3] FIG. 3 is a graph showing the relative value (viability) of mitochondrial activity in Example 1.
[FIG. 4] FIG. 4 is a graph showing the relative value of the number of cells in Example 1.
[FIGs. 5A and 5B] FIGs. 5A and 5B are photographs showing the results of alkaline phosphatase staining in Example 1.
[FIG. 6] FIG. 6 shows graphs showing the staining results by the anti-TRA-1-60 in Example 1.
[FIG. 7] FIG. 7 shows graphs showing the staining results by the anti-SSEA-4 in Example 1.

### DESCRIPTION OF EMBODIMENTS

### <Composition for Undifferentiated State Maintenance Culture>

As described above, the composition for pluripotent cell undifferentiated state maintenance culture of the present invention includes microbubbles. The maintenance composition of the present invention is characterized by having microbubbles, and other configurations and conditions are not particularly limited. According to the maintenance composition of the present invention, pluripotent cells can be cultured in an undifferentiated state.

As a result of their intensive study, the inventors of the present invention have found that by including microbubbles the growth rate of pluripotent cells can be improved or the frequency of cellular death of pluripotent cells can be reduced in the pluripotent cell maintenance culture, thereby establishing the present invention. According to the present invention, since the growth rate of pluripotent cells can be improved or the frequency of cellular death of pluripotent cells can be reduced in the pluripotent cell maintenance culture, the pluripotent cell maintenance culture can be performed while maintaining the cells in an undifferentiated state more efficiently than a composition for pluripotent cell undifferentiated state maintenance culture.

As used herein, "pluripotent" means having the ability to differentiate into three germ layers, including, for example, endoderm, mesoderm, and ectoderm. As used herein, "pluripotent" also includes, for example, having the ability to differentiate into all cells, i.e., totipotency. The pluripotent cells are not particularly limited, and examples thereof include embryonic stem cells (ES cells), induced pluripotent stem cells (iPS cells), nuclear transfer ES cells (ntES cells), germ stem cells, somatic stem cells, and embryonic tumor cells; iPS cells are preferable. The origin of the pluripotent cell is not particularly limited, and examples thereof include humans and non-human animals. Examples of non-human animals include primates such as monkeys, gorillas, chimpanzees, and marmosets; mice; rats; dogs; rabbits; sheep; horses; and guinea pigs.

As used herein, "differentiation ability" means that, for example, when pluripotent cells are cultured under a condition of differentiating into target differentiated cells, some or all of the pluripotent cells can be differentiated into the target differentiated cells.

As used herein, "undifferentiated" means, for example, that the differentiation ability is not changed or that pluripotent cells are not differentiated before and after culturing.

As used herein, "pluripotent cell undifferentiated state maintenance" means, for example, that pluripotent cells are maintained in an undifferentiated state. Specifically, "pluripotent cell undifferentiated state maintenance" means, for example, that pluripotent cells are in a state of maintaining the differentiation ability, i.e., the differentiation ability to three germ layers. Whether pluripotent cells maintain an undifferentiated state can be evaluated based on, for example, a marker such as a cell surface marker in cells obtained by performing maintenance culture of target pluripotent cells. Specifically, when the pluripotent cells are iPS cells, the cell surface marker may be Stage-Specific Embryonic Antigen-4 (SSEA-4), TRA-1-60, or the like. TRA-1-60 is, for example, keratan sulfate (sugar chain) on cell-surface localized podocalyxin (glycoprotein). Therefore, when SSEA-4 or TRA-1-60 is expressed in the cells after the maintenance culture, that is, when the cells are positive for SSEA-4 or TRA-1-60, the pluripotent cells can be evaluated as maintaining an undifferentiated state after the maintenance culture, for example. On the other hand, when neither SSEA-4 nor TRA-1-60 is expressed in the cells after the maintenance culture, that is, when the cells are negative for SSEA-4 and TRA-1-60, the pluripotent cells can be evaluated as not maintaining an undifferentiated state after the maintenance culture, for example. The expression of the cell surface marker can be evaluated by flow cytometry according to Example 1 described below. As the marker, for example, alkaline phosphatase activity may be used. When the pluripotent cells are iPS cells, for example, SSEA-3, octamer-binding transcription factor 4 (OCT4), SRY (sex determining region Y)-box 2 (SOX2), NANOG, TRA-1-81, or the like may be used as the cell surface marker. TRA-1-81 is, for example, keratan sulfate (sugar chain) on cell-surface localized podocalyxin (glycoprotein).

As used herein, "pluripotent cell undifferentiated state maintenance culture" means culturing such that the undifferentiated state of pluripotent cells is maintained after the maintenance culture, for example. In the present invention, for example, when pluripotent cells are included after the maintenance culture, the maintenance culture can be evaluated as the pluripotent cell undifferentiated state maintenance culture. In addition, as used herein, the "pluripotent cell undifferentiated state maintenance culture" may be evaluated based on, for example, the number of pluripotent cells before and after the maintenance culture. In this case, if the number is equal to (no significant difference) or more than the number of cells before the maintenance culture, the maintenance culture can be evaluated as, for example, the pluripotent cell undifferentiated state maintenance culture. The "pluripotent cell undifferentiated state maintenance culture" may be evaluated, for example, based on the proportion of the markerexpressing cells after the maintenance culture. Specifically, in the case where the marker is TRA-1-60, when the proportion of TRA-1-60 positive cells in the cells after the iPS cell culture is, for example, 10% or more, 20% or more, 30% or more, 40% or more, 50% or more, 60% or more, 70% or more, 80% or more, 85% or more, 90% or more, 95% or more, 96% or more, 97% or more, 98% or more, or 99% or more, the maintenance culture can be evaluated as, for example, the pluripotent cell undifferentiated state maintenance culture. In the case where the marker is SSEA-4, when the proportion of SSEA-4 positive cells in the cells after the iPS cell culture is, for example, 10% or more, 20% or more, 30% or more, 40% or more, 50% or more, 60% or more, 70% or more, 80% or more, 85% or more, 90% or more, 95% or more, 96% or more, 97% or more, 98% or more, or 99% or more, the maintenance culture can be evaluated as, for example, the pluripotent cell undifferentiated state maintenance culture.

The "positive (+)" means that a high signal is detected by an analysis method such as flow cytometry detected using an antigen-antibody reaction as compared to a negative control reaction using negative control cells that do not express the antigen or antibodies that do not react with the antigen. The "negative (-)" means that an equivalent or lower signal is detected by an analysis method such as flow cytometry detected using an antigen-antibody reaction as compared to a negative control reaction using negative control cells that do not express the antigen or antibodies that do not react with the antigen.

As used herein, "microbubble" means a closed minute space made of a gas surrounded by something other than the gas, and can also be referred to as, for example, a minute bubble. The microbubble may be, for example, a fine bubble. "Fine bubble" generally means a microbubble having a bubble diameter of less than 100 µm. "Bubble diameter" means a spherical equivalent diameter of the bubble. The bubble diameter may be a mean diameter (arithmetic mean diameter) of microbubbles obtained by the measurement method to be described below. The fine bubble may be a microbubble (FB) or an ultrafine bubble (UFB). A microbubble generally has a bubble diameter of 1 µm or more and less than 100 µm. An ultrafine bubble generally is a microbubble having a bubble diameter of less than 1 µm. Preferably, the microbubble is surrounded by an aqueous solvent, i.e., the gas component is surrounded by an aqueous medium with the aqueous medium being contacted at an outer periphery of the microbubble.

The microbubbles are present dispersed in a medium. The microbubbles are present dispersed in whole or in part in the medium. In the latter case, the microbubbles may be localized to a part of the medium. The medium can be, for example, a liquid or solid. Examples of the liquid include an aqueous solvent including water, an oily solvent, and a mixed solvent thereof. Further, the liquid includes a sol. Examples of the solid include solids obtained by coagulating the liquid. Further, the solid includes a gel. Examples of the liquid include physiological saline; a buffer such as a phosphate buffer; an infusion such as an extracellular solution, an intracellular solution, or the like; water such as distilled water, pure water, or the like; a pluripotent cell maintenance medium (culture solution); a cell medium (culture solution); and an organ-storage solution. The solid may be, for example, a solidified matter of the liquid. The pluripotent cell maintenance medium may be a self-prepared medium or a commercially available medium, and specific examples thereof include Cellartis^{®} DEF-CS 500 Basal Medium (produced by Cellartis), StemFit^{®} (produced by Ajinomoto Co., Inc.), mTeSR-1 (produced by STEMCELL), TeSR-E8 (produced by STEMCELL Technologies Inc.), StemPRO hESC SFM (produced by Life Technologies), E8 (produced by Life Technologies), and ReproStem (produced by REPROCELL Inc.). Examples of the cell-culture solution include media based on Dulbecco's Modified Eagle's Medium (DMEM), Minimal essential Medium (MEM), Basal Medium Eagle (BME), RPMI1640, Ham's F-10, Ham's F-12, α Minimal essential Medium (αMEM), Glasgow's Minimal essential Medium (GMEM), and Iscove's Modified Dulbecco's Medium (IMDM).

The microbubble includes any gas as a gas (gas component). Specific examples of the microbubble include biogas such as carbon monoxide (CO), hydrogen sulfide (H₂S), hydrogen (H₂), and the like; rare gases such as helium (He), argon (Ar), krypton (Kr), xenon (Xe), and the like; oxygen (O₂); carbon dioxide (CO₂); nitrous oxide (N₂O); carbon dioxide (CO₂); nitrogen (N₂); methane (CH₄); ethane (CH₃CH₃); propane (CH₃CH₂CH₃); fluoromethane (CH₃F); difluoromethane (CH₂F₂); carbon tetrafluoride (CF₄); ethylene oxide (C₂H₄O); and air. The microbubble excludes the case where the gas is air-only, for example. As used herein, the "air" means, for example, air (atmosphere) used in producing the microbubbles. It is preferable that the gas in the microbubbles be a gas derived from a medical gas when it has a medical gas grade.

The microbubble density means the number of microbubbles relative to the volume of the medium. The "density" can also be referred to as a number concentration. The lower limit of the microbubble density is, for example, 5×10⁵ bubbles/ml, 1×10⁶ bubbles/ml, 5×10⁶ bubbles/ml, 1×10⁷ bubbles/ml, 5×10⁷ bubbles/ml, 1×10⁸ bubbles/ml, 5×10⁸ bubbles/ml, 1×10⁹ bubbles/ml, preferably 1×10⁶ bubbles/ml, 5×10⁶ bubbles/ml, 1×10⁷ bubbles/ml, 5×10⁷ bubbles/ml, 1×10⁸ bubbles/ml, or 5×10⁸ bubbles/ml. The upper limit of the microbubble density is, for example, 1.5×10⁹ bubbles/ml, 2×10⁹ bubbles/ml, 3×10⁹ bubbles/ml, 5×10⁹ bubbles/ml, 7×10⁹ bubbles/ml, 9×10⁹ bubbles/ml, 1×10¹⁰ bubbles/ml, 5×10¹⁰ bubbles/ml, 1×10¹¹ bubbles/ml, 5×10¹¹ bubbles/ml, 1×10¹² bubbles/ml, or 5×10¹² bubbles/ml. The microbubble density is in the range, for example, from 5×10⁵ bubbles/ml to 5×10¹² bubbles/ml, 5×10⁵ bubbles/ml to 1×10¹² bubbles/ml, 5×10⁵ bubbles/ml to 5×10¹¹ bubbles/ml, 5×10⁵ bubbles/ml to 1×10¹¹ bubbles/ml, 5×10⁵ bubbles/ml to 5×10¹⁰ bubbles/ml, 5×10⁵ bubbles/ml to 1×10¹⁰ bubbles/ml, 1×10⁶ bubbles/ml to 9×10⁹ bubbles/ml, 5×10⁶ bubbles/ml to 9×10⁹ bubbles/ml, 1×10⁷ bubbles/ml to 7×10⁹ bubbles/ml, 5×10⁷ bubbles/ml to 7×10⁹ bubbles/ml, 1×10⁸ bubbles/ml to 5×10⁹ bubbles/ml, 5×10⁸ bubbles/ml to 5×10⁹ bubbles/ml, 1×10⁹ bubbles/ml to 3×10⁹ bubbles/ml, 5×10⁸ bubbles/ml to 2×10⁹ bubbles/ml, or 5×10⁸ bubbles/ml to 1.5×10⁹ bubbles/ml.

The density, bubble diameter, and mean diameter (hereinafter also referred to as "characteristics") of the microbubbles can be appropriately measured according to the medium in which the microbubbles are dispersed. When the microbubbles are dispersed in a liquid medium, the characteristics of the microbubbles can be calculated by analyzing the bubbles in the maintenance composition of the present invention by a particle-trajectory-analysis method. The particle-trajectory-analysis method can be performed using, for example, NanoSight^{®} NS300 (produced by Malvern Instruments) in accordance with example 1, to be described below. The characteristics of the microbubbles may be calculated by an analysis method other than the particle-trajectory-analysis method. In this case, the characteristics of the microbubbles obtained by the other analysis method satisfy the above-mentioned exemplification when converted into the calculation value obtained by the particle-trajectory-analysis method. When the microbubbles are dispersed in a solid medium, the characteristics of the microbubbles can be calculated based on the characteristics of the microbubbles in the liquid before solidification of the medium and the characteristics of the microbubbles in the liquid obtained by dissolving the solid medium.

When the microbubble contains oxygen as a gas component, the proportion of O₂ in the gas is, for example, greater than 0%, 100% or less, 10% to 100%, 20% to 100%, 30% to 100%, 40% to 100%, 50% to 100%, 60% to 100%, 70% to 100%, 80% to 100%, 90% to 100%, 95% to 100%, 96% to 100%, 97% to 100%, 98% to 100%, or 99% to 100%.

The maintenance composition of the present invention may further include a pluripotent cell undifferentiated state maintenance factor, for example. The undifferentiated state maintenance factor is, for example, a factor added to maintain the undifferentiated state of pluripotent cells in the pluripotent cell maintenance culture. Examples of the undifferentiated state maintenance factor include interleukin (IL)-3, IL-6, stem cell factor (SCF), thrombopoietin (TPO), FMS-like tyrosine kinase 3 ligand (Flt-3L), leukemia inhibitory factor (LIF), chemokine (C-C motif) ligand 2 (CCL2), basic fibroblast growth factor (bFGF), transforming growth factor-β(TGF-β), NODAL, and insulin. In the present invention, the amount of the undifferentiated state maintenance factor is not particularly limited as long as it does not interfere with the function of the microbubbles.

The maintenance composition of the present invention may include other components. The other components may include, for example, a component contained in a cell culture solution, and specific examples thereof include salts such as sodium, potassium, calcium, magnesium, phosphorus, chlorine, and the like; growth factors or hematopoietic growth factor such as cytokines, chemokines, fibroblast growth factors, epidermal growth factors, vascular endothelial growth factors, platelet-derived growth factors, hepatocyte growth factors, and the like; vitamins such as vitamin B1 and vitamin C; sera such as fetal calf serum, human serum, horse serum, chicken serum, and the like; lipids such as cholesterol and the like; sugars such as lactoferrin and the like; reducing agents such as monothioglycerol, 2-mercaptoethanol, and the like; and other additives such as ethanolamine, amino acids, sodium pyruvate, selenium sodium acid, and the like. In the present invention, the content of the other components is not particularly limited as long as it does not interfere with the function of the microbubbles.

The maintenance composition of the present invention can be produced, for example, by a method for producing microbubbles such as fine bubbles using a gas. Thus, a method for producing the maintenance composition of the present invention includes, for example, producing microbubbles using a gas and a medium. As a specific example, when the maintenance composition of the present invention is a liquid, the liquid composition can be produced by using, for example, a gas, the medium, and a microbubble-production apparatus of a swirling flow type, an ejector type, a venturi type, a static mixer type, a micropore type, a pressure dissolution type, or an ultrasonic cavitation type. In addition, when the maintenance composition of the present invention is a solid, the solid composition can be produced by coagulating the liquid composition by a known method. When the solid is a gel, the gel composition can be produced, for example, by mixing the liquid composition with a gelling agent. At the start of the producing microbubbles, the state of the gas may be a gas, a liquid, or a solid. The gas may include a plurality of types of gases. In this case, each gas may be separately subjected to the producing microbubbles, or all or some of the gases may be subjected to the producing microbubbles at the same time. When the maintenance composition of the present invention includes an undifferentiated state maintenance factor and/or another component, the undifferentiated state maintenance factor and/or other component may be added before, during, or after the producing bubbles.

The maintenance composition of the present invention may be used, for example, *in vivo* or *in vitro.* The maintenance composition of the present invention can also be used, for example, as a reagent for research, and can be used for producing a pharmaceutical or a cell preparation.

The maintenance composition of the present invention can efficiently perform pluripotent cell maintenance culture while maintaining the cells in an undifferentiated state. Thus, the maintenance composition of the present invention can be suitably used, for example, as a pluripotent cell maintenance culture medium (culture solution).

### < Medium for Undifferentiated State Maintenance Culture>

As described above, the medium for pluripotent cell undifferentiated state maintenance culture of the present invention includes the composition for pluripotent cell undifferentiated state maintenance culture of the present invention. The maintenance medium of the present invention is characterized in that it includes the maintenance composition of the present invention, and other configurations and conditions are not particularly limited. According to the maintenance medium of the present invention, the pluripotent cell maintenance culture can be performed while maintaining the cells in an undifferentiated state more efficiently than it can using the medium for pluripotent cell undifferentiated state maintenance culture that includes substantially no microbubbles. Regarding the maintenance medium of the present invention, reference can be made to the description as to the maintenance composition of the present invention.

The maintenance medium of the present invention may be, for example, a solid or a liquid. When the medium of the present invention is a liquid, the maintenance medium of the present invention may be, for example, a maintenance culture solution.

The maintenance medium of the present invention preferably includes, for example, the maintenance composition and the pluripotent cell maintenance medium of the present invention, and more preferably includes the maintenance composition, the pluripotent cell maintenance medium, and the undifferentiated state maintenance factor of the present invention.

When the maintenance medium of the present invention includes a plurality of components, each component may be stored in a separate container. In this case, the maintenance medium of the present invention may be referred to as, for example, a maintenance medium kit. In this case, the maintenance medium kit of the present invention is used by mixing the components, for example, at the time of use.

### <Maintenance Culture Method>

As described above, the method for pluripotent cell maintenance culture of the present invention includes pluripotent cell maintenance culturing in the presence of microbubbles. The maintenance culture method of the present invention is characterized by pluripotent cell maintenance culturing in the presence of microbubbles, and other steps and conditions are not particularly limited. According to the maintenance culture method of the present invention, the pluripotent cell maintenance culture can be performed while maintaining the cells in an undifferentiated state more efficiently than it can when the condition of the pluripotent cell undifferentiated state maintenance culture includes substantially no microbubbles. Regarding the maintenance culture method of the present invention, reference can be made to the description as to the maintenance composition and the maintenance medium of the present invention.

The pluripotent cells may be self-prepared cells or commercially available cells, for example. When the pluripotent cells are self-prepared cells, the maintenance culture method of the present invention may induce pluripotent cells to be subjected to the maintenance culture prior to the maintenance culturing. In this case, the maintenance culture method of the present invention includes inducing pluripotent cells. The pluripotent cells that can be induced are not particularly limited, and examples thereof include embryonic stem cells (ES cells), induced pluripotent stem cells (iPS cells), nuclear transfer ES cells (ntES cells), germ stem cells, somatic stem cells, and embryonic tumor cells; ES cells or iPS cells are preferable.

The method for inducing pluripotent cells is not particularly limited, and pluripotent cells may be induced by isolating them from, for example, living bodies, organs, human organs, tissues, or fertilized eggs, or by introducing reprogramming factors into cells.

As a specific example, when the pluripotent cells are iPS cells, the following method can be used.

The inducing can be conducted, for example, by culturing cells (target cells) into which iPS cells are induced after contacting with the reprogramming factors. Specifically, the inducing is conducted, for example, in the presence of a medium (culture solution). The medium may be, for example, a medium corresponding to the type of the cells or a pluripotent cell maintenance medium described above. The medium is preferably replaced, for example, periodically (e.g., every one or two days) after the start of the culture. The contacting can be performed, for example, by introducing the reprogramming factor into the target cell, and, for example, lipofection, electroporation, microinjection, or a method for introducing a nucleic acid such as viral vector or a protein into a cell can be used.

The target cells are not particularly limited, and examples thereof include prenatal, newborn, or adult-derived somatic cells or healthy subject or patient-derived somatic cells; cultured cells such as primary-cultured cells, passage cells, cell lines, and the like; tissue stem cells (somatic stem cells) such as neural stem cells, hematopoietic stem cells, mesenchymal stem cells, dental pulp stem cells, and the like; progenitor cells; and differentiated cells of lymphoid cells, epithelial cells, endothelial cells, muscle cells, fibroblast cells (skin cells, etc.), hair cells, hepatic cells, gastric mucosal cells, enterocytic cells, splenic cells, pancreatic cells, brain cells, lung cells, renal cells, fat cells, and the like. Regarding the origin of the target cell, for example, reference can be made to the above description as to the origin of the pluripotent cell.

The target cell density at the start of the inducing is not particularly limited, and, when culturing in a cell culture vessel such as a plate, the density is, for example, 1×10¹ to 1×10⁵ cells/cm², 1×10¹ to 5×10⁴ cells/cm², and preferably 4×10⁴ to 5×10⁴ cells/cm².

Examples of the reprogramming factor include a gene specifically expressed in ES cells, a gene product or non-coding RNA thereof, a gene related to ES cell undifferentiated state maintenance, a gene product or non-coding RNA thereof, and a small-molecule compound having similar effect. Examples of the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. The genes that can serve as the reprogramming factors may be used alone or in combination. Regarding the combination, for example, reference can be made to WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, and the like.

The culture conditions in the inducing are not particularly limited. The culture temperature is, for example, 30°C to 40°C. The CO₂ concentration during the culture may be, for example, 2% to 10%. The pH of the medium is, for example, 6.5 to 7.8, preferably 7 to 7.8 or 7.2 to 7.6. The culture is preferably performed in a humid environment. As a specific example, the culture conditions are as follows. That is, for example, the culture temperature is about 37°C, the CO₂ concentration is about 5%, and the environment is a humid environment. The number of culture days in the culturing is, for example, 10 to 30 days. Thus, in the inducing, for example, ES cell-like colonies can be generated after the culture.

The inducing may be conducted, for example, in the absence of feeder cells or in the presence of feeder cells (e.g., on feeder cells). In the latter case, the feeder cells are preferably feeder cells that have been treated so as not to proliferate in advance. As a specific example, the feeder cells are preferably feeder cells treated with a growth inhibitor such as mitomycin C. Examples of feeder cells include fibroblasts such as embryonic fibroblasts (including mouse embryonic fibroblasts, STO cells, SNL cells, SL10 cells, and the like), and stromal cells such as PA6 cells (mouse stromal cell lines), MS-5 cells, OP9 cells, and the like. The feeder cells may be, for example, cells that are not in contact with the reprogramming factor among the cells to be subjected to the inducing. In this case, regarding the inducing, for example, reference can be made to the description of WO 2010/137746.

The inducing may be conducted, for example, under hypoxic condition. The hypoxic condition is, for example, that the O₂ concentration is 15% or less, and preferably 0.1% to 15%. In this case, regarding the inducing, for example, reference can be made to the description of WO 2010/013845.

In the maintenance culture method, for example, the iPS cells may be selected after the inducing. The selection may be performed, for example, by using the above-described markers, for example. Specifically, the selection may be performed based on the shapes of colonies formed, or may be performed by using the expression of a marker gene (such as Oct3, Oct4, or Nanog) induced by reprogramming or a marker linked to the expression of the gene, or may be performed by using the activity of alkaline phosphatase as an indicator.

As another specific example, when the pluripotent cells are ES cells, the following example can be given.

The inducing can be conducted, for example, by culturing a cell mass isolated from a blastocyst of an animal. As a specific example, regarding the method for inducing ES cells, reference can be made to the following Reference 1. Regarding the origin of the target cells, for example, reference can be made to the above description as to the origin of pluripotent cells. Reference 1: Thomson JA et al., "Embryonic stem cell lines derived from human blastocysts," Science, 1998, vol. 282, pages 1145-1147.

Regarding the culture conditions in the inducing, for example, reference can be made to the description as to the culture conditions in the inducing of iPS cells.

In the maintenance culture method, for example, the ES cells may be selected after the inducing. The selection may be performed, for example, by using the above-described markers, for example. Specifically, the selection may be performed based on the shapes of colonies formed, or may be performed by using the expression of a marker gene (such as Oct3, Oct4, Nanog, or ECAD) of ES cells or a marker linked to the expression of the gene, or may be performed by using the activity of alkaline phosphatase as an indicator.

Next, in the maintenance culturing, the pluripotent cell maintenance culture is performed in the presence of microbubbles. Specifically, the maintenance culturing can be conducted by, for example, performing the pluripotent cell maintenance culture in the presence of a medium (culture solution) containing microbubbles. In this case, the microbubbles are contained in the medium. Thus, for example, the maintenance composition or the maintenance medium of the present invention may be used as the medium. The medium is preferably replaced, for example, periodically (e.g., every one or two days) after the start of the culture. The medium replacement is a replacement of a part of or the whole medium during the culture.

In the maintenance culturing, the microbubbles are present during the entire or a part of the period of the maintenance culture and are present preferably during the entire period of the maintenance culture. In the maintenance culturing, when the microbubbles are present in a part of the period, the period in which the microbubbles are present is, for example, 50% or more, 60% or more, 70% or more, 80% or more, 85% or more, 90% or more, 95% or more, 96% or more, 97% or more, 98% or more, 99% or more, or 100%, based on the entire period of the maintenance culturing. Regarding the density and properties of the microbubble in the maintenance culturing, for example, reference can be made to the above description.

When the microbubbles are present during the entire period of the maintenance culturing, a medium including the microbubbles may be used at the start of the maintenance culturing, or the maintenance composition or the maintenance medium of the present invention may be added at the start of the maintenance culturing. When the microbubbles are present during the entire period of the maintenance culturing, the medium is replaced with a medium including the microbubbles. The microbubble density is, for example, the microbubble density in the medium to be added or the medium immediately after the addition at the start of the maintenance culturing and/or at the time of the medium replacement.

When the microbubbles are present during a part of the period of the maintenance culturing, a medium including the microbubbles may be used at the start of the maintenance culturing, or the maintenance composition or the maintenance medium of the present invention may be added at the start or after the start of the maintenance culturing. When the microbubbles are present during a part of the period of the maintenance culturing, the medium may be replaced with a medium including the microbubbles or may be replaced with a medium including substantially no microbubbles. As used herein, "including substantially no microbubbles" means, for example, that the microbubble density in the medium is less than or equal to the detection limit. The microbubble density is, for example, the microbubble density in the medium including the microbubbles added or the medium including the microbubbles immediately after the addition at the start of the maintenance culturing and/or at the time of the medium replacement.

The pluripotent cell density at the start of the maintenance culturing is not particularly limited, and, when culturing in a cell culture vessel such as a plate, the density is, for example, 1×10¹ to 1×10⁵ cells/cm², 1×10¹ to 5×10⁵ cells/cm², and preferably 1×10⁵ to 5×10⁵ cells/cm².

The maintenance culturing may be started by subjecting the pluripotent cells obtained in the inducing as it is, or may be started by recovering the pluripotent cells obtained in the inducing or the pluripotent cells during the passage and seeding them, for example. In the latter case, the pluripotent cells may be separated into single cells and seeded, or the recovered cell mass may be seeded, for example. When separating into the single cells, the single cells can be prepared, for example, by treating the recovered pluripotent cells with a protease, a calcium chelator, or the like. Examples of the protease include trypsin and collagenase. When the single cells are seeded, the maintenance culturing is preferably conducted in the presence of a ROCK inhibitor, for example, because the viability of the pluripotent cells can be improved. Examples of the ROCK inhibitor include Y-27632 (CAS No.: 146986-50-7), Fasudil/HA1077 (CAS No.: 105628-07-7), H-1152 (CAS No.: 871543-07-6), and WF-5362 (CAS No.: 539857-64-2).

The culture conditions in the maintenance culturing are not particularly limited, and general conditions in the pluripotent cell maintenance culture can be adopted. As a specific example, the culture temperature is, for example, 30°C to 40°C. The CO₂ concentration during the culture may be, for example, 2% to 10%. The pH is, for example, 6.5 to 7.8, and preferably 7 to 7.8 or 7.2 to 7.6. The culture is preferably performed in a humid environment. As a specific example, the culture conditions are as follows. That is, for example, the culture temperature is about 37°C, the CO₂ concentration is about 5%, and the environment is a humid environment. The number of culture days in the maintenance culturing is, for example, one to 10 days.

The maintenance culturing may be conducted, for example, in the absence of feeder cells or in the presence of feeder cells (e.g., on feeder cells). In the latter case, the feeder cells are preferably feeder cells that have been treated so as not to proliferate in advance. As a specific example, the feeder cells are preferably feeder cells treated with a growth inhibitor such as mitomycin C. Regarding the feeder cells, reference can be made to the above description.

When the maintenance culturing is conducted in the absence of feeder cells, the maintenance culturing is preferably conducted in the presence of an extracellular matrix. In this case, the maintenance culturing can be conducted by using, for example, a cell culture instrument coated with the extracellular matrix. The extracellular matrix means, for example, a molecule that serves as a scaffold for a cell.

Examples of the extracellular matrix include elastin; entactin; collagens such as collagen type I, collagen type II, collagen type III, collagen type IV, collagen type V; collagen type VII, and the like; tenascin; fibrillin; fibronectin; laminin; vitronectin; proteoglycans composed of sulfated glucosaminoglycans such as chondroitin sulfate, heparan sulfate, keratan sulfate, and dermatan sulfate, and core proteins; glucosaminoglycans such as chondroitin sulfate, heparan sulfate, keratan sulfate, dermatan sulfate, hyaluronic acid, and the like; and Synthemax^{®} (vitronectin derivative); and Matrigel^{®} (mixture of laminin, collagen type IV, heparin sulfate proteoglycans, entactin/nidogen, etc.), and laminin is preferable. Examples of the laminin include laminin 111, laminin 121, laminin 211, laminin 213, laminin 222, laminin 311 (laminin 3A11), laminin 332 (laminin 3A32), laminin 321 (laminin 3A21), laminin 3B32, laminin 411, laminin 421, laminin 423, laminin 521, laminin 522, and laminin 523. The three numbers in each laminin are the names of the constituent subunits of the α chain, the β chain, and the γ chain from the left, respectively. As a specific example, laminin 111 is composed of an α1 chain, a β1 chain, and a γ1 chain. The laminin 3A11 is composed of an α3A chain, a β1 chain, and a γ1 chain. The cell culture substrate may include a peptide fragment of the protein or a fragment of the sugar chain. As a specific example, the peptide fragment of the protein may be a fragment of laminin. Examples of the fragment of laminin include the above-described fragments of laminin, and specific examples thereof include laminin 211-E8, laminin 311-E8, laminin 411-E8, and laminin 511-E8. The laminin 211-E8 is composed of fragments of an α2 chain, a β1 chain, and a γ1 chain of laminin. The laminin 311-E8 is composed of fragments of an α3 chain, a β1 chain, and a γ1 chain of laminin. The laminin 411-E8 is composed of fragments of an α4 chain, a β1 chain, and a γ1 chain of laminin. The laminin 511-E8 is composed of, for example, fragments of an α5 chain, a β1 chain, and a γ1 chain of laminin. The laminin fragment is a fragment having integrin binding activity.

In order to maintain the undifferentiated state of the pluripotent cells, it is preferable that the maintenance culturing is conducted in the presence of the undifferentiated state maintenance factor. Regarding the undifferentiated state maintenance factor, for example, reference can be made to the above description.

In the maintenance culture method of the present invention, the maintenance culturing may be conducted once or a plurality of times. When the maintenance culturing is conducted a plurality of times, pluripotent cells may be passaged (passaging) between the maintenance culturing. The passaging can be performed, for example, by recovering cells from a cell culture instrument or the like after the maintenance culturing and seeding pluripotent cells in a new cell culture instrument.

In the passaging, pluripotent cells may be evaluated or selected from the recovered cells and subjected to passaging. The evaluation can be made, for example, by using the marker described above. Regarding the selection, for example, reference can be made to the description as to the selection in the inducing.

The maintenance culture method of the present invention may further include, for example, inducing differentiation of target differentiated cells from pluripotent cells that have been maintenance-cultured. Regarding the differentiated cells, reference can be made to the above description. The method for inducing the differentiated cells from the pluripotent cells can be appropriately performed according to, for example, the type of the differentiated cells.

According to the maintenance culture method of the present invention, pluripotent cell maintenance culture can be performed while maintaining the cells in an undifferentiated state more efficiently than the condition of the pluripotent cell undifferentiated state maintenance culture including substantially no microbubbles.

### <Pluripotent Cell Production Method>

As described above, the method for producing pluripotent cells of the present invention includes producing pluripotent cells by performing pluripotent cell maintenance culture in an undifferentiated state, wherein the producing is conducted by the method for pluripotent cell undifferentiated state maintenance culture according to the present invention. The production method of the present invention is characterized in that the producing is conducted by the maintenance culture method of the present invention, and other steps and conditions are not particularly limited. According to the production method of the present invention, pluripotent cell maintenance culture can be performed while maintaining the cells in an undifferentiated state more efficiently than the condition of the pluripotent cell undifferentiated state maintenance culture including substantially no microbubbles. Therefore, according to the production method of the present invention, pluripotent cells can be produced efficiently. Regarding the production method of the present invention, reference can be made to the descriptions as to the maintenance composition, the maintenance medium, and the maintenance culture method of the present invention.

### <Use of Composition or Medium>

The present invention provides a composition or use thereof for use in pluripotent cell undifferentiated state maintenance culture, wherein the composition includes microbubbles. The present invention also provides a medium or use thereof for use in pluripotent cell undifferentiated state maintenance culture, wherein the composition includes microbubbles. Regarding the use of the present invention, reference can be made to the descriptions as to the maintenance composition, maintenance medium, maintenance culture method, and production method of the present invention.

### Examples

The examples of the present invention are described below. The present invention, however, is not limited by the following examples.

### Example 1

It was examined that the maintenance composition of the present invention can perform pluripotent cell maintenance culture while maintaining the cells in an undifferentiated state more efficiently than a pluripotent cell undifferentiated state maintenance culture composition including substantially no microbubbles.

### (1) Material

iPS cells (human iPS cell line 12 (ChiPSC12), produced by Cellartis) were used as pluripotent cells, and a medium kit (Cellartis^{®} DEF-CS^{™} 500 Culture System, produced by Cellartis) capable of performing maintenance culture in the absence of feeder cells was used as a pluripotent cell maintenance medium. The medium kit includes Cellartis DEF-CS 500 Basal Medium (medium), Cellartis DEF-CS 500 COAT-1 (plate-coating agent), and Cellartis DEF-CS 500 Additives (medium additives, DEF-CS GF-1, DEF-CS GF-2, and DEF-CS GF-3). When the frozen pluripotent cells were thawed and passaged, the above-described medium kit was mixed with the culture medium composition 1 described below, and then heated at 37°C for about 30 minutes to use (passage medium). As will be described below, in the maintenance medium of Examples, a composition containing O₂ as a bubble component is added to use. Specifically, after introducing microbubbles into Cellartis DEF-CS 500 Basal Medium by the method to be described in Example 1(2), a maintenance culture medium and a replacement medium (maintenance medium) were prepared using a medium including microbubbles.

### (Medium Composition 1)

| | |
|---|---|
| Cellartis DEF-CS 500 Basal Medium | 10 ml |
| DEF-CS GF-1 | 30 µl |
| DEF-CS GF-2 | 10 µl |
| DEF-CS GF-3 | 10 µl |
| Total | 10.05 ml |

When replacing the medium during the pluripotent cell maintenance culture, the medium kit was mixed with the medium composition 2 described below, and then heated at 37°C for about 30 minutes to use (replacement medium).

### (Medium Composition 2)

| | |
|---|---|
| Cellartis DEF-CS 500 Basal Medium | 10 ml |
| DEF-CS GF-1 | 30 µl |
| DEF-CS GF-2 | 10 µl |
| Total | 10.04 ml |

### (2) Production of Maintenance Composition

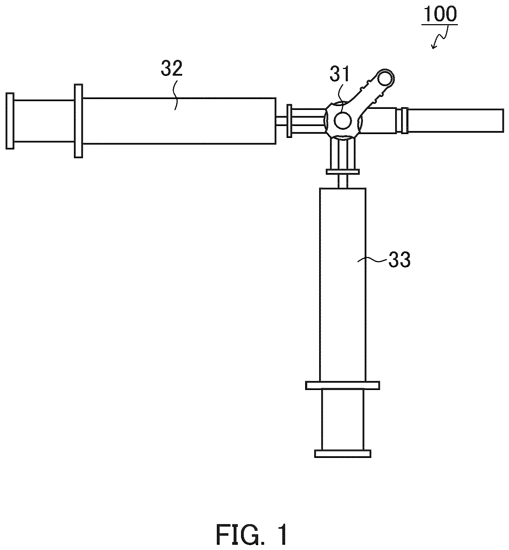

The maintenance compositon of the present invention was prepared using a microbubble production apparatus 100 shown in FIG. 1. As shown in FIG. 1, the production apparatus 100 includes syringes 32 and 33 disposed on two sides of a three-way stopcock 31. In the production apparatus 100, the syringes 32 and 33 communicate with each other via the three-way stopcock 31. First, the syringe 32 was released from the three-way stopcock 31, and 20 ml of Cellartis DEF-CS 500 Basal Medium was introduced into the inside thereof. Next, the syringe 32 was again connected to the three-way stopcock 31 to remove the gas in the three-way stopcock 31. After the removal, the syringe 33 was released from the three-way stopcock 31 to introduce 20 ml of oxygen (O₂ concentration: 99.999(v/v)%) into the inside thereof. Then, the syringe 33 was again connected to the three-way stopcock 31. After the connection, microbubbles containing O₂ as a gas component were produced by continuously reciprocating each plunger of the syringes 32 and 33 in each outer cylinder of the syringes 32 and 33 for 10 minutes, thereby producing the maintenance compound of Example 1. Specifically, the medium and the oxygen were introduced to the syringes so that the volume ratio of the medium to the oxygen was 1:1. After the introduction, the syringe was sealed with a three-way stopcock, inverted and mixed, and allowed to stand at room temperature (about 25°C) for one hour. Next, the gas in the syringe was replaced with fresh oxygen, and then the syringe was inverted and mixed and allowed to stand at room temperature for another four hours, thereby preparing the maintenance composition of Reference Example 1. Then, the maintenance culture medium and the replacement medium (maintenance medium) of Reference Example 1 were prepared using the maintenance composition of Reference Example 1.

The physical properties of the obtained maintenance composition of Example 1 were measured using a NanoSight^{®} NS300 (produced by Malvern Instrument) after standing for about one hour with default parameters. The measurement was performed at 25°C. As a result, the microbubble mean diameter and the microbubble density were as described below. While a small amount of a medium additive was added to the obtained composition to prepare a passage medium and a replacement medium, the amount of the additive is substantially negligible. Thus, the maintenance compositions of Example 1 below are the microbubble mean diameter and microbubble density in the maintenance culture medium and the replacement medium (maintenance medium).

### (Maintenance Compositions of Example 1)

Mean diameter: 162.1 ± 12.5 nm, Density: 2.94 × 10⁹ ± 6.63 × 10⁸ bubbles/ml

### (3) Passage of Pluripotent Cell

First, Cellartis DEF-CS COAT diluted solution was added to a 96-well dish (for MTT assay, produced by Sarstedt), a 24-well dish (for alkaline phosphatase assay, produced by Sarstedt), or a 12-well dish (for counting the number of cells and flow cytometry, produced by Sarstedt), and incubated at 37°C for 20 minutes or more to coat the wells of the dishes. The diluted solution was prepared by diluting DEF-CS COAT-1 20-fold with Dulbecco's PBS containing Ca&Mg (D-PBS +/+) (Cat No.: C-40230, produced by PromoCell GmbH).

Next, the medium was removed from a T75 flask in which the iPS cells have been cultured, and then washed once with 1 ml of D-PBS (-/-) (Cat No.: 166-23555, produced by FUJIFII,M Wako Pure Chemical Corporation). After the washing, 1.5 ml of a tryptic solution (Cat. No.: 12563-029, produced by TrypLE Select, Life Technologies) was added. The tryptic solution was spread over the entire surface of iPS cells and then allowed to stand at 37°C for four to five minutes. After the degree of peel of iPS cells was checked by microscopy, iPS cells were peeled by tapping. After the peeling, 13.5 ml of a passage medium was added to the T75 flask to suspend the cells. After recovering the mixture of the trypsin solution and the passage medium, a part of the mixture was collected and stained with trypan blue, and the number of cells was counted using a cell counter (CountessII, produced by Thermo Fisher Scientific).

The iPS cells after counting were seeded to the dishes being coated so as to achieve 4.0 × 10⁴ cells/cm². The medium at the time of seeding was used for the passage medium. After the seeding, the cells were incubated at 37°C in a humid environment of 5% CO₂.

### (4) Pluripotent Cell Maintenance Culture

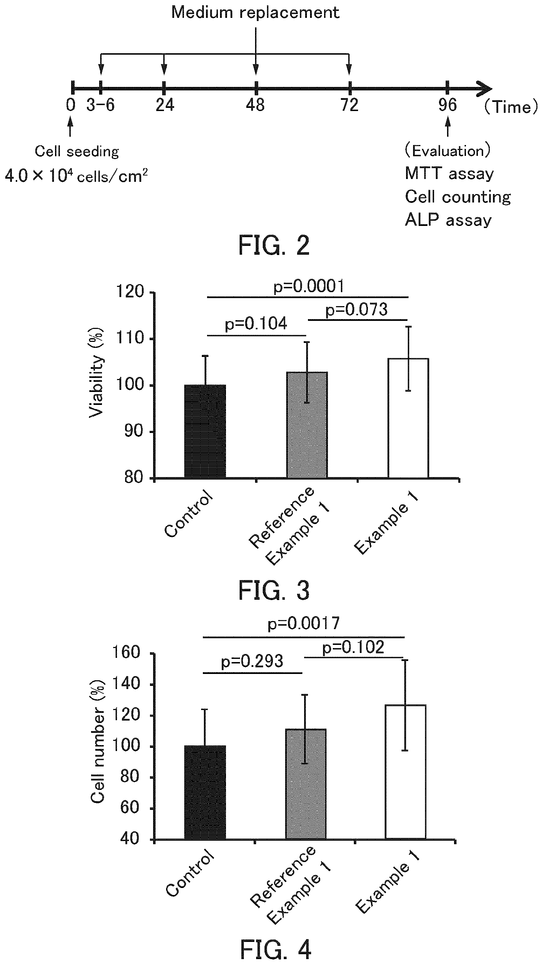

Next, as shown in FIG. 2, the culture medium of each well was replaced with the maintenance medium of Example 1 or the maintenance medium of Reference Example 1 at three to six hours, at one day (24 hours), two days (48 hours), and three days (72 hours) after the start of the culture.

Then, the cells in the respective wells were recovered at 96 hours after the start of the culture and subjected to counting of the number of cells, MTT assay, or flow cytometry. The 24-well dish was subjected to an alkaline phosphatase assay as it was. The recovery of the cells after the culture was performed in the same manner as the recovery at the time of passage.

### (5) MTT assay

The MTT assay was performed using a MTT Assay Kit (CellTiter 96^{®} AQueous One Solution Cell Proliferation Assay (MTS), Cat. No.: G358A, produced by Promega Corporation) according to the protocol provided. As a control, the MTT assay was performed in the same manner except that a replacement medium including no microbubbles was used instead of the maintenance medium of Example 1 or Reference Example 1. Then, the relative value of the mitochondrial activity was calculated with the measured value of the control being 100%. Similar tests were performed a total of five times and the results of the mean values are shown in FIG. 3. The statistical processing was performed using the One-way ANOVA (hereinafter, the same applies).

FIG. 3 is a graph showing the relative value (viability) of the mitochondrial activity. In FIG. 3, the horizontal axis indicates the type of maintenance medium and the vertical axis indicates the relative value of the mitochondrial activity. As shown in FIG. 3, when the cells were cultured in the maintenance medium of Example 1, the relative value of the mitochondrial activity was significantly increased compared to the maintenance medium of the control and the maintenance medium of Reference Example 1. That is, it was found that the maintenance medium of Example 1 improved the viability of iPS cells in the iPS cell maintenance culture.

### (6) Cell Number Counting

The number of cells recovered from each well was counted using the cell counter. As a control, the number of cells was counted in the same manner except that the replacement medium was used instead of the maintenance medium of Example 1 or Reference Example 1. Then, the relative value of the number of cells was calculated with the number of cells of the control being 100%. Similar tests were performed a total of three times and the results of the mean values are shown in FIG. 4.

FIG. 4 is a graph showing the relative value of the number of cells. In FIG. 4, the horizontal axis indicates the type of maintenance medium and the vertical axis indicates the relative value of the number of cells. As shown in FIG. 4, when the cells were cultured in the maintenance medium of Example 1, the relative value of the number of cells was significantly increased as compared with the maintenance medium of the control and the maintenance medium of Reference Example 1. When compared with the improvement rate of the viability of (3), the increase rate of the number of iPS cells when cultured in the maintenance medium of Example 1 was large. From this, it was found that the maintenance medium of Example 1 improved the proliferation efficiency of iPS cells in the iPS cell maintenance culture.

### (7) Alkaline Phosphatase Assay

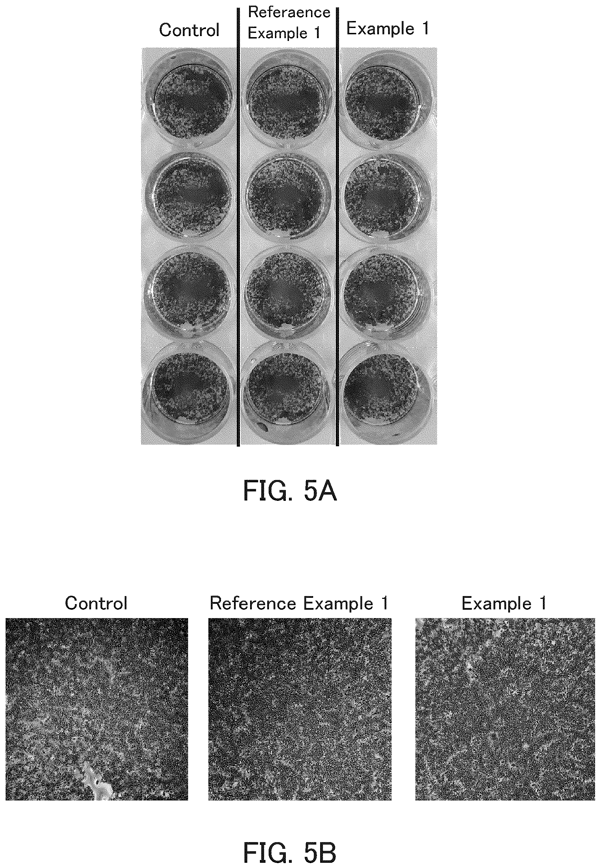

The alkaline phosphatase assay was performed on a 24-well dish using an alkaline phosphatase assay kit (TRACP & ALP double-stain Kit, Cat. No.: MK300, produced by TAKARABIO INC.) according to the protocol provided. As a control, the alkaline phosphatase assay was performed in the same manner except that the replacement medium was used instead of the maintenance medium of Example 1 or Reference Example 1. The results are shown in FIGs. 5A and 5B.

FIGs. 5A and 5B show photographs showing the results of alkaline phosphatase staining. FIG. 5A shows photographs of the entire dish, and FIG. 5B shows enlarged photographs inside the dish. As shown in FIGs. 5A and 5B, the alkaline phosphatase activity was observed in iPS cells cultured in the maintenance medium of Example 1 in the same manner as that of iPS cells cultured in the other maintenance medium including the control.

### (8) Cell Surface Marker

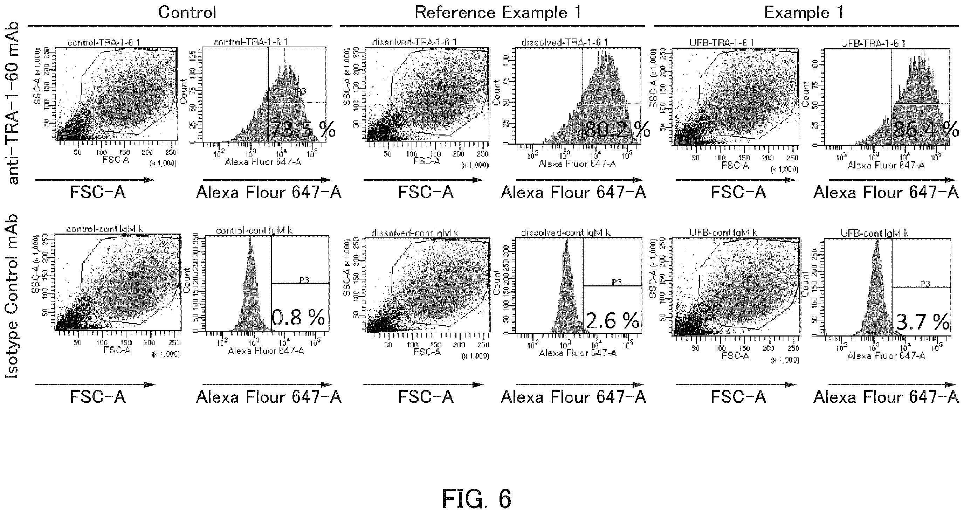

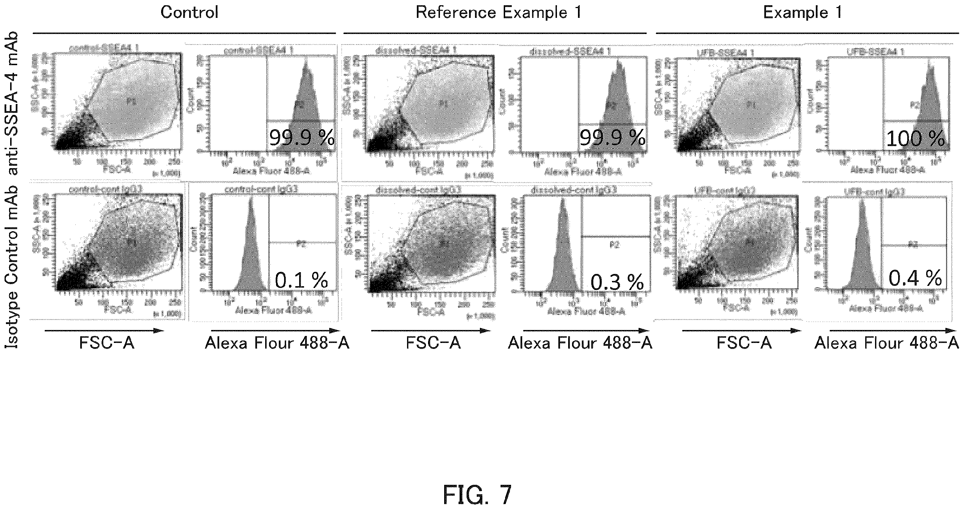

The recovered cells were stained with an anti-SSEA-4 (Human/Mouse SSEA4 Fluorescein conjugated Monoclonal Antibody, Cat. No.: FAB1435F, produced by R&D Systems) or an isotype control antibody (Mouse IgG3 Fluorescein-conjugated Antibody, Cat. No.: IC007F, produced by R&D Systems) and an anti-TRA-1-60 (Alexa Fluor^{®} 647 Mouse anti-Human TRA-1-60 Antigen, BD Pharmingen^{™}, Cat. No.: 560850) or an isotype control antibody (Alexa Fluor^{®} 647 Mouse IgM, κ Isotype Control, BD Pharmingen^{™}, Cat. No.: 560806). After the staining, the expression of each marker was measured on the obtained cells using a flow cytometer (FACSCanto II, produced by BD Biosciences). As a control, the staining and measurement were performed in the same manner, except that the replacement medium was used instead of the maintenance medium of Example 1 or Reference Example 1. These results are shown in FIGs. 6 and 7.

FIG. 6 shows the staining results of the anti-TRA-1-60, and FIG. 7 shows the staining results of the anti-SSEA-4. In FIGs. 6 and 7, the horizontal axis indicates the FSC-A or the fluorescent intensity (Alexa Flour-488 or 647), the vertical axis indicates the SSC-A or the cell count, and the numerical values in the graphs indicate the proportion of cells that are TRA-1-60 positive or SSEA-4 positive. As shown in FIGs. 6 and 7, it was verified that iPS cells obtained after culturing in the maintenance medium of Example 1 were SSEA-4 positive and TRA-1-60 positive as in the case of iPS cells cultured in the other maintenance medium including the control, and the proportion of positive cells was equivalent to the case of iPS cells cultured in the other maintenance medium including the control. That is, it was verified that iPS cells after culturing in the maintenance medium of Example 1 maintained an undifferentiated state.

From the above, it was found that the maintenance composition of the present invention can perform pluripotent cell maintenance culture in an undifferentiated state more efficiently than the medium for pluripotent cell undifferentiated state maintenance culture including substantially no microbubbles. It was also found that the efficient maintenance culture was caused by improvement of the viability and proliferation efficiency of pluripotent cells.

While the present invention has been described above with reference to illustrative embodiments and examples, the present invention is by no means limited thereto. Various changes and variations that may become apparent to those skilled in the art may be made in the configuration and specifics of the present invention without departing from the scope of the present invention.

This application claims priority to Japanese Patent Application No. 2020-123653, filed on July 20, 2020, the entire disclosure of which is incorporated herein by reference.

### (Supplementary Notes)

Some or all of the above example embodiments and examples may be described as in the following Supplementary Notes, but are not limited thereto.

### (Supplementary Note 1)

A composition for pluripotent cell undifferentiated state maintenance culture, including:
microbubbles.

### (Supplementary Note 2)

The composition according to Supplementary Note 1, wherein
the microbubbles contain, as a gas component, at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).

### (Supplementary Note 3)

The composition according to Supplementary Note 1 or 2, wherein
the microbubbles contain oxygen as a gas component.

### (Supplementary Note 4)

The composition according to Supplementary Note 1, wherein
the gas component is surrounded by an aqueous solvent.

### (Supplementary Note 5)

The composition according to any one of Supplementary Notes 1 to 4, wherein
a microbubble density is 5×10⁵ bubbles/ml to 5×10¹² bubbles/ml.

### (Supplementary Note 6)

The composition according to any one of Supplementary Notes 1 to 5, further including:
a medium, wherein
the medium is at least one of a liquid or a solid.

### (Supplementary Note 7)

The composition according to any one of Supplementary Notes 1 to 6, wherein
the pluripotent cells are embryonic stem cells (ES cells) or induced pluripotent stem cells (iPS cells).

### (Supplementary Note 8)

A medium for pluripotent cell undifferentiated state maintenance culture, including:
the composition according to any one of Supplementary Notes 1 to 7.

### (Supplementary Note 9)

A method for pluripotent cell undifferentiated state maintenance culture, including:
pluripotent cell maintenance culturing in the presence of microbubbles.

### (Supplementary Note 10)

The method according to Supplementary Note 9, wherein
the microbubbles contain, as a gas component, at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).

### (Supplementary Note 11)

The method according to Supplementary Note 9 or 10, wherein
the microbubbles contain oxygen as a gas component.

### (Supplementary Note 12)

The method according to any one of Supplementary Notes 9 to 11, wherein
a microbubble density is 5×10⁵ bubbles/ml to 5×10¹² bubbles/ml.

### (Supplementary Note 13)

The method according to any one of Supplementary Notes 9 to 12, wherein
the pluripotent cells are ES cells or iPS cells.

### (Supplementary Note 14)

A method for producing pluripotent cells, including:
producing pluripotent cells by performing pluripotent cell maintenance culture in an undifferentiated state, wherein
the producing is conducted by the method according to any one of Supplementary Notes 9 to 13.

### (Supplementary Note 15)

A composition for use in pluripotent cell undifferentiated state maintenance culture, wherein the composition includes microbubbles.

### (Supplementary Note 16)

A medium for use in pluripotent cell undifferentiated state maintenance culture, wherein
the medium includes microbubbles.

As described above, according to the present invention, the growth rate of pluripotent cells can be improved or the frequency of cellular death of pluripotent cells can be reduced, as compared to a composition for pluripotent cell undifferentiated state maintenance culture including substantially no microbubbles. Therefore, the present invention is extremely useful, for example, in the field of regenerative medicine and the like.

## Claims

1. A composition for pluripotent cell undifferentiated state maintenance culture, comprising:
microbubbles.

2. The composition according to claim 1, wherein
the microbubbles contain, as a gas component, at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).

3. The composition according to claim 1 or 2, wherein
the microbubbles contain oxygen as a gas component.

4. The composition according to any one of claims 1 to 3, wherein
a microbubble density is 5×10⁵ bubbles/ml to 5×10¹² bubbles/ml.

5. The composition according to any one of claims 1 to 4, further comprising:
a medium, wherein
the medium is at least one of a liquid or a solid.

6. The composition according to any one of claims 1 to 5, wherein
the pluripotent cells are embryonic stem cells (ES cells) or induced pluripotent stem cells (iPS cells).

7. A medium for pluripotent cell undifferentiated state maintenance culture, comprising:
the composition according to any one of claims 1 to 6.

8. A method for pluripotent cell undifferentiated state maintenance culture, comprising:
pluripotent cell maintenance culturing in the presence of microbubbles.

9. The method according to claim 8, wherein
the microbubbles contain, as a gas component, at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).

10. The method according to claim 8 or 9, wherein
the microbubbles contain oxygen as a gas component.

11. The method according to any one of claims 8 to 10, wherein
a microbubble density is 5×10⁵ bubbles/ml to 5×10¹² bubbles/ml.

12. The method according to any one of claims 8 to 11, wherein
the pluripotent cells are ES cells or iPS cells.

13. A method for producing pluripotent cells, comprising:
producing pluripotent cells by performing pluripotent cell maintenance culture in an undifferentiated state, wherein
the producing is conducted by the method according to any one of claims 8 to 12.
